# EUROPEAN PATENT APPLICATION

(11) **EP 3 932 943 A1**
(43) Date of publication of application: **05.01.2022**
(21) Application number: 19917055.6
(22) Date of filing: 26.12.2019
(51) Int. Cl.: C07K 14/59, A61K 38/24, C12N 15/16, C12N 15/64, A61P 5/34

(54) **BIOLOGICALLY ACTIVE RECOMBINANT EQUINE CHORIONIC GONADOTROPIN (RECG) AND PROCESS FOR OBTAINING SAME, VETERINARY COMPOSITION AND USE**

(30) Priority: 28.02.2019 BR 102019004147
(71) Applicant: Ouro Fino Saúde Animal Participações S.A., CEP : 14140-000 Cravinhos SP (BR); Universidade de São Paulo - USP, 05508-220 São Paulo (BR)
(72) Inventor: SOGAYAR, Mari Cleide, 05586-090 São Paulo (BR); OLIVEIRA CARREIRA NISHIYAMA, Ana Cláudia, 05539-080 São Paulo (BR); ALMEIDA DEMASI, Marcos Angelo, 04581-050 São Paulo (BR); CAMARGO, Lauren, 05360-130 São Paulo (BR); MALDONADO COELHO, Tatiane, 04313-002 São Paulo (BR)
(74) Representative: Clarke Modet & Co.
(86) International application number: PCT/BR2019/050564
(87) International publication number: WO 2020/172728

(57) **Abstract**

The present invention relates to the production of a biologically active recombinant equine chorionic gonadotropin (reCG), a veterinary composition comprising said reCG, and the use of said reCG or a composition comprising said reCG. The reCG of the present invention has a glycosylation profile similar to that of commercially available chorionic gonadotropins and exhibits an *in vivo* activity level corresponding to the eCG and similar to that of FSH and LH hormones. The present invention finds use in the field of Biotechnology, more specifically, in the field of Assisted Reproduction in animals, being especially suitable for use in the treatment of veterinary conditions related to mammal reproduction and ovulation, as well as for the manufacture of diagnostic kits or as a cell culture supplement.

## Description

### Technical Field

The present invention relates to an improved process for the production of biologically active recombinant equine chorionic gonadotropin, referred to herein as reCG or reCGa+β, the reCG produced using the process of the present invention, a veterinary composition comprising reCG, and the use of reCG or the composition comprising reCG.

The produced reCG has a glycosylation profile similar to that of commercially available chorionic gonadotropins and exhibits an *in vivo* activity level similar to that of Luteinizing Hormone (LH) and Follicle Stimulating Hormone (FSH).

The present invention finds use in the field of biotechnology, more specifically, in the field of assisted reproduction in animals, being especially suitable for use in the treatment of veterinary conditions related to mammal reproduction and ovulation, for the manufacture of diagnostic kits or as a cell culture supplement.

### History of the invention

Currently, Brazil is in a privileged position as the world's largest producer and exporter of beef, making livestock one of the most important and profitable economic activities in the country. This data shows the importance of basic and technological research in cattle reproduction, especially in the field of ovulation stimulating hormones, such as equine chorionic gonadotropin (eCG).

Several hormones are used in techniques for assisted reproduction in animals and, in recent years, equine chorionic gonadotropin has emerged as the first option to induce ovulation in gestational anestrus cows, in timed artificial insemination (TAI) protocols, in order to improve the fertility indices of cows reared in an extensive management system (SÁ FILHO et al., Equine chorionic gonadotropin and gonadotropin-releasing hormone enhance fertility in a norgestomet-based, timed artificial insemination protocol in suckled Nelore (Bos Indicus) cows. Theriogenology 2010; 73(5):651-8; FERREIRA et al., Effect of different doses of equine chorionic gonadotropin on follicular and luteal dynamics and P/Al of high-producing Holstein cows. Anim Reprod Sci 2013; 140(1-2) 26-33; CARVALHO et al. Equine chorionic gonadotropin improves the efficacy of a timed artificial insemination protocol in buffalo during the nonbreeding season. Theriogenology 2013;79(3):423-8; BARREIROS et al., Dynamics of follicular growth and progesterone concentrations in cyclic and anestrous suckling Nelore cows (Bos indicus) treated with progesterone, equine chorionic gonadotropin, or temporary calf removal. Theriogenology 2014; 81(5):651-6).

eCG is especially used in beef cows raised in an extensive management system and has also been used in superovulation treatments of cows and heifers as an effective treatment option in irascible animals (MAPLETOFT et al., Recent advances in the superovulation in cattle. Reprod Nutr Dev 2002; 42 (6): 601-11), whereas conventional hormonal treatments in cattle use eight doses of commercial preparations of purified porcine FSH - Folltropin-V^{©}' Bioniche^{©} (data on the product package sheet).

The eCG is secreted by the equine uterus, more specifically by the endometrial calyces, which are formed around the forty-fifth day of gestation, remaining until around the eighty-fifth day of gestation. Secretion of this hormone, which has a biological action similar to both FSH and LH, stimulates the development of ovarian follicles in the pregnant mare. Some of these follicles ovulate, but most become luteinized follicles due to their LH-like activity. Such accessory corpora lutea produce the progesterone required to maintain pregnancy in the mare (HAFEZ & HAFEZ, Animal Reproduction. Publisher Manole 2004; single volume).

The eCG currently used in animal reproduction is purified from the plasma of pregnant mares (CHRISTAKOS & BAHL, Pregnant mare serum gonadotropin. Purification and physicochemical, biological, and immunological characterization. J Biol Chem 1979; 254(10):4253-61; MAPLETOFT et al., Recent advances in the superovulation in cattle. Reprod Nutr Dev 2002; 42(6):601-11), being marketed as Folligon (Intervet); SyncroeCG (Ourofino) and Novormon (Coopers), and there is no commercially available recombinant eCG.

In heterologous species this unique gonadotropin has the ability to bind to both FSH and LH receptors and to stimulate folliculogenesis (STEWART et al., Pregnant mare serum gonadotrophin: ratio of follicle-stimulating hormone and luteinizing hormone activities measured by radioreceptor assay. J Endocrinol 1976; 71(3):471-82; COMBARNOUS, Original hypothesis on the specificity of the binding of glycoprotein hormones to their receptors. C R Seances Acad Sci III 1981). Its prolonged plasma half-life due to its high sugar content (MCINTOSH et al., Pregnant mare serum gonadotrophin: rate of clearance from the circulation of sheep. J Reprod Fertil 1975; 44(1):95-100), allows that a single application of this hormone be efficient to induce ovulation, thus saving time on handling, and even reducing possible stresses to animals undergoing superovulation treatments (KIMURA et al., Successful superovulation of cattle by a single administration of FSH in aluminum hydroxide gel. Theriogenology 2007; 68(4):633-9).

In addition to the fact that extraction of gonadotropins is a laborious and expensive task, lack of biosafety of the resulting material is of great concern, since any purification attempt that preserves glycoprotein hormones shows limited capacity to destroy resistant viruses (THARASANIT et al., Effects of recombinant human follicle stimulating hormone on follicle development and ovulation in the mare. Theriogenology 2006; 65(6):1071-81). Also, for being a biological extract, there is great variability in glycoprotein activity between different eCG batches, hence contributing to the variable response and quality of the obtained embryos (WILSON et al., Superovulation of cattle with a recombinant-DNA bovine follicle hormone. Animal Reproduction Science 1993;33(1-4):71-82).

In view of the foregoing and taking into account the fact that the origin source of eCG is limited (blood of pregnant mares) and also that bioethical factors related to obtaining this material exist, there is a need for an unlimited, ethical source of eCG that is more practical to obtain and reproducible, ensuring an uninterrupted supply of this hormone to a growing consumer market.

Recombinant gonadotropins exhibit maximum biosafety, better performance in results, no interference from other hormones and few contaminants, being, therefore, widely used in human Reproductive Medicine, being of great interest for assisted reproduction in animals. Recombinant technology has proven that it is possible to produce complex proteins in a reproducible manner. However, the structure of gonadotropins is difficult to reproduce, since in addition to comprising two independently transcribed and translated chains, several post-translational modifications exist, in particular glycosylation, which in the case of eCG plays a key role in its biological activity (LOUMAGEL et al., Human follicle-stimulating hormone produced by recombinant DNA technology: a review for clinicians. Human Reproduction 1995; v. 1 p. 188-199; MARTINUK et al., Effects of carbohydrates on the pharmacokinetics and biological activity of equine chorionic gonadotropin in vivo. Biol Reprod. 1991 Oct; 45(4):598-604).

Over the last decades, numerous processes have been developed to allow the genetic manipulation of cultured cells in such a way that these cells started to produce a large variety of proteins and glycoproteins of pharmaceutical interest. These processes involve the use of recombinant DNA technology to prepare expression vectors containing genetic material that encodes a particular protein or glycoprotein. When the expression vector is introduced into a certain cell line in culture, said genetic material starts to instruct the cellular biosynthetic machinery in such a way that the synthesis of a specific protein or glycoprotein occurs.

However, there are several obstacles associated with the production of glycoproteins in a given genetically modified cell line. The glycosylation process associated with the production of glycoproteins consists of the addition of carbohydrates to the polypeptide chains, which is a post-translational event that results in the addition of sugar chains to specific amino acid residues of asparagine (N-linked glycosylation) or serine/ threonine (O-linked glycosylation) on a particular polypeptide sequence. In a glycoprotein, while the polypeptide moiety is encoded by a certain genetic sequence, thus being constant, the carbohydrate structures are variable, a feature that is known as micro- and macro-heterogeinity. The glycosylation process is the result of a complex sequence of enzymatic reactions that add, cleave and modify the carbohydrate moiety of a glycoprotein. Anticipating the result of the coordinated activity of these enzymes that participate in the glycosylation process is neither intuitive nor a direct activity, and it is very difficult to predict which glycosylation profiles can be obtained from a particular cell type, or even how fluctuations in the gene expression profile or manipulations in culture regimes can affect the final glycosylation profile (Al-Rubeai, Cell Engineering. Springer 2009; pp. 247-279). Therefore, several cell lines commonly used for the heterologous production of proteins of therapeutic interest have variable abilities to promote glycoprotein glycosylation. However, although these cells have such capabilities, the cellular glycosylation machinery is not controlled by the expression vector of the protein of interest. Thus, the glycoprotein produced by a certain genetically engineered cell line may have a structure of glycans different from that of the natural glycoprotein encoded by the expression vector.

Polysaccharide chains modulate the biological activity of glycoproteins in several ways, promoting, for example: a) a reduction in the plasma *"clearance"* with a resulting increased half-life time; b) mediation of molecular recognition processes; c) stabilization of the polypeptide chain conformation; and d) protection against proteolytic degradation (Al-Rubeai, Cell Engineering. Springer 2009; pages 247-279).

Like other gonadotropins, eCG is a heterodimeric glycoprotein composed of two polypeptide subunits, α and β chains. In equines, luteinizing hormone (eLH) and eCG have identical polypeptide chains, and the divergence in the N- and O-glycan structure dictates the difference between the molecular weights and biological activity of these two hormones: eCG exhibits exceptional circulatory half-life as compared to eLH (BOUSFIELD et al., Identification of twelve O-glycosylation sites in equine chorionic gonadotropin beta and equine luteinizing hormone ss by solid-phase Edman degradation. Biol Reprod. 2001 Jan;64(1):136-47). The state of the art teaches that removal of terminal sialic acid residues from eCG glycan chains dramatically decreases the *in vivo* activity of this glycoprotein, as its half-life is reduced from six days to approximately 1 to 2 hours (MARTINUK et al., Effects of carbohydrates on the pharmacokinetics and biological activity of equine chorionic gonadotropin in vivo. Biol Reprod. 1991 Oct; 45(4):598-604). eCG α subunit, which comprises 96 amino acids, exhibits two complex N-linked oligosaccharide chains located at asparagin residues 56 and 82. However, the carbohydrate chains of these two hormones, eLH and eCG, exhibit differences in their structures: eLH subunitα has two biantennary N-glycans terminated in sulfated N-acetylgalactosamines (SO4-4-GaINAc), while the N-glycans attached to eCG α subunit are terminated in sialic acid at α2,3 and α2,6 linkages (DAMM et al., Structure determination of the major N- and O-linked carbohydrate chains of the beta subunit from equine chorionic gonadotropin. Eur J Biochem. 1990 Apr 20;189(1):175-83.), possibly extended with lactosamine repeats (BOUSFIELD et al., Differential effects of alpha subunit Asparagine56 oligosaccharide structure on equine lutropin and follitropin hybrid conformation and receptor-binding activity. Biochemistry. 2004 Aug 24;43(33):10817-33). The eCG β subunit, composed of 149 amino acid residues (SUGINO et al., Structural studies on equine glycoprotein hormones. Amino acid sequence of equine chorionic gonadotropin beta-subunit. J Biol Chem. 1987 Jun 25;262(18):8603-9), has a single N-glycan chain located at Asn13, terminating with sulfated GalNAc in eLH and sialylated a2,3Gal in eCG (SMITH et al., Equine lutropin and chorionic gonadotropin bear oligosaccharides terminating with SO4-4-GalNAc and Sia alpha 2,3Gal, respectively. J Biol Chem. 1993 Jan 15;268(2):795-802; MATSUI et al., Structural analysis of N-linked oligosaccharides of equine chorionic gonadotropin and lutropin beta-subunits. Biochemistry. 1994 Nov 29;33(47): 14039-48). Both β subunits have up to 12 O-glycans extended with sialylated poly N-acetyl-lactosamine (a2,3) located at serines or threonines of the carboxy-terminal peptide (CTP) of 28 amino acid residues (HOKKE et al., Structure determination of the disialylated poly-(N-acetyllactosamine)-containing O-linked carbohydrate chains of equine chorionic gonadotropin. Glycoconj J. 1994 Feb;11(1):35-41; BOUSFIELD et al., Identification of twelve O-glycosylation sites in equine chorionic gonadotropin beta and equine luteinizing hormone ss by solid-phase Edman degradation. Biol Reprod. 2001 Jan;64(1):136-47).

In the literature, several authors have tried to obtain recombinant equine chorionic gonadotropin, mainly an artificial variant of reCG, consisting of a β- and α- chain fusion protein, which was cloned and expressed in insect cells (LEGARDINIER et al., Biological activities of recombinant equine luteinizing hormone/chorionic gonadotropin (eLH/CG) expressed in Sf9 and Mimic insect cell lines. Journal of Molecular Endocrinology 2005; 34:47-60 ); secreted in milk from transgenic rabbits (GALET et al., Expression of an in vitro biologically active equine LH/CG without C-terminal peptide (CTP) and/or beta26-110 disulphide bridge. J Endocrinol, 2001; 167(1):117-24); expressed in mouse cells (GALET et al., Expression of a single beta-alpha chain protein of equine LH/CG in milk of transgenic rabbits and its biological activity. Mol Cell Endocrinol, 2001; 174(1-2):31-40); and modified and expressed as deglycosylated forms in CHO cells (Chinese Hamster Ovary cells). Only for the first two of the aforementioned systems *in vivo* biological activity assays were performed, resulting in no eCG-like activity, which fact was probably caused by the difference in the glycan chains added to eCG by these expression systems.

Moreover, the state of the art teaches methods of producing recombinant gonadotropins, but it fails to propose an efficient way to obtain reCG in large amounts while having a glycosylation profile similar to wild-type eCG to ensure reCG *in vivo* activity similar to that observed for wild-type eCG.

HESSER (HESSER, A survey of heterologous expression systems for the production of bovine follicle stimulating hormone and luteinizing hormone. All Dissertations 2011; Paper 692) describes the production of gonadotropins, more specifically, of follicle-stimulating hormones in CHO cells.

FURUHASHI *et al.* (FURUHASHI et al., Fusing the carboxyterminal peptide of the chorionic gonadotropin (CG) betasubunit to the common alphasubunit:retention of O-linked glycosylation and enhanced in vivo bioactivity of chimeric human CG. Mol Endocrinol. 1995 Jan;9(1):5463) describes an hCG analog having a CTP (Carboxy Terminal Peptide)-like polypeptide tail and lists the importance of glycosylation in the biological activity of the analog.

SUGAHARA *et al.* (SUGAHARA et al., Biosynthesis of a biological active single peptide chain containing the human common α and chorionic gonadotropin β subunits in tandem. Proc Natl Acad Sci USA 1995; 92:2041-2045) describes the biological activity of a single, biologically active polypeptide chain containing a hCG β-subunit fused to gonadotropin α-subunit.

Later, SUGAHARA *et al.* (SUGAHARA et al., Expression of biological active fusion genes encoding the common alpha subunit and the follicle-stimulating hormone beta subunit: Role of a linker sequence. J Biol Chem 1996; 271:10445-10448) described the conversion of a FSH heterodimer into a single-chain structure, and the impact of the absence of the linker sequence on secretion and binding rates of said protein. Furthermore, it shows that alignment of α and β domains differ from that presented in the form of heterodimer.

NARAYAN *et al.* (NARAYAN et al., Functional expression of yoked human chorionic gonadotropin in baculovirus-infected insect cells. Mol Endocrinol. 1995; 9(12):1720-6) describes the simultaneous expression of eCG α- and β-subunits in insect cells, whose expression is controlled by different promoters in the same recombinant baculovirus, generating proteins that are biologically active *in vitro* only.

SHIOTA *et al.* (SHIOTA et al., Production of recombinant eCG with potent FSH-like activity by site-directed mutagenesis. Nippon Yakurigaku Zasshi 1997; 110(1):59P-62P) describes recombinant gonadotropins having different structures in view of the coupled oligosaccharides and relates the biological activity of mutants or variants.

EP0974599 describes a recombinant eCG variant and its corresponding DNA sequence, as well as a pharmaceutical composition of said recombinant eCG for use as a drug for animals. The disclosed recombinant eCG variant has eCG α- and β- chains fused into a single polypeptide chain, having FSH- and LH-like activity *in vitro* only. The inventors suggest its use as a medicine for animals, but do not demonstrate: a) the similarity between the glycosylation profile of the obtained recombinant form and that of the wild-type form; and b) the *in vivo* biological activity of the obtained recombinant eCG form.

Document US 5,767,251 describes methods to generate recombinant human gonadotropins, including hCG, hLH and hFSH, wherein hormones composed of two different subunits are synthesized in the same cell by at least one expression vector, in which expression of each subunit is controlled by a different promoter. In this instance, the hCG biological activity is easily differentiated from the LH activity because, unlike equines, in humans, the β chains of these two gonadotropins are encoded by different genes.

US 5,047,335 describes a process for controlling the glycosylation of recombinant proteins involving the use of CHO cells genetically engineered to produce sialyltransferases, a class of glycosyltransferases. Such additional production of sialyltransferases allows, for example, that recombinant glycoproteins produced in this genetically engineered CHO cell line, have a glycan structure closer to natural human glycoproteins. However, document US 5,047,335 is silent on which combination of glycosyltransferases would be required for one to achieve recombinant eCG having biological activity.

US 6,103,501, US 6,238,890 and US 6,987,172 describe artificial single-chain gonadotropin variants which are naturally found as heterodimers, such as, for example, CG, TSH, LH and FSH, and which can provide effects or functions, or may behave as agonists or antagonists of native hormones. Also in this instance, human CG biological activity is easily differentiated from human LH activity because, unlike equines, in humans the β chains of these two gonadotropins are encoded by different genes. Furthermore, these documents do not describe what conditions would be required for one to obtain recombinant eCG having biological activity.

US 2010/120677 describes methods for the production of recombinant biologically active eFSH analogs and methods for improving mammal reproduction using recombinant eFSH analogs.

Document CA 2183564 describes methods for increasing fertility by reducing the activity or levels of hormones having LH activity, and methods for selecting antibodies to specific moieties of certain proteins, including LH and hCG, to reduce their biological activity.

Document WO2016178087A1 describes methods for the production of unmodified eCG forms, or eCG variants fused to the Fc portion ("Fragment crystallizable region") in the milk of transgenic animals but fails to demonstrate the *in vivo* biological activity of the eCG forms thus produced.

The above state of the art shows the constant efforts directed towards the development of new recombinant gonadotropins and processes to produce them with quality in a safe and unlimited manner, further emphasizing the need for new solutions and the importance of Research & Development in this field.

There remains, therefore, the need for improvements in the process of producing recombinant eCG, so that it is produced in large amounts whereas having the glycosylation profile required for its biological activity *in vivo.*

Thus, based on this knowledge, the present invention provided an improvement in methods for the production of recombinant eCG, ensuring the production of large amounts of recombinant eCG having a glycosylation profile similar to that of commercially available chorionic gonadotropins and, accordingly, having *in vivo* biological activity also similar to that of wild-type eCG. Such recombinant chorionic gonadotropins are produced by selecting specific cell phenotypes from a biotechnologically prepared system for high expression of these structures.

### Summary of the invention

Although many recombinant heterodimeric gonadotropins have been successfully produced by using molecular and cell biology techniques, an *in vivo* biologically active form of recombinant eCG has not yet been achieved. Moreover, it is unknown which heterologous expression system based on mammalian cells is able to promote N-linked and O-linked glycosylation post-translational modifications of recombinant eCG in large amounts, which are required to distinguish the eCG biological activity from that of eLH.

In mares, wild-type eCG is produced by a differentiated and highly specialized cell type, the endometrial calyx cells, and also, at a very particular time in these animals' lives, that is, between the forty and eighty-fifth days of gestation. Details on molecular events associated with the promotion of eCG post-translational modifications, particularly glycosylation processes, which are essential for its biological activity, are unknown. Thus, it is not possible to precisely anticipate which cell types, conditions and factors would be associated with the achievement of such glycosylation profile compatible with an eCG-like biological activity, highlighting: 1) which heterologous expression system would mimic the combination of enzymes and factors found in endometrial calyx cells; or 2) what are the conditions for generating a heterologous system and what are the growth conditions of the heterologous system associated with the achievement of a reCG glycosylation profile compatible with *in vivo* biological activity.

The present invention concerns an improved process for the production of a biologically active recombinant equine chorionic gonadotropin, in which α and β polypeptide chains are produced in the same cell, said reCG having a glycosylation profile similar to that of commercially available chorionic gonadotropins. Said improved process is based on the process described in co-owned document PCT/BR2015/050047.

Thus, the Applicant has surprisingly found that by using widely known molecular and cell biology techniques combined with the use of appropriate phenotypic selection strategies, it is possible to select cell line sub-phenotypes in culture, which are associated with a glycosylation profile similar to that found in wild-type eCG, thus ensuring that the recombinant eCG thus obtained has *in vivo* biological activities similar to FSH and LH hormones.

Such technical effect is even more unexpected since, so far, the prior art has taught that such activity could only be obtained if at least 50% of the terminal branches of eCG glycan chains were sialylated (MARTINUK et al., Effects of carbohydrates on the pharmacokinetics and biological activity of equine chorionic gonadotropin in vivo. Biol Reprod. 1991 Oct; 45(4):598-604). More specifically, the reCG achieved by the improved process can be fully glycosylated, being characterized by containing N- and O-linked glycan chains having high level of terminal sialylation of galactose residues, or even being partially glycosylated, containing Gal-(1-4)GIcNAc disaccharide unit as N-linked and/or O-linked terminal residue, or the GlcNAc monosaccharide unit as an O-linked terminal residue.

According to a first aspect, a process is provided which comprises the steps of:
(a) preparing and obtaining DNA sequences encoding reCG α and β chains;
(b) inserting said coding DNA sequences into expression vectors such that said coding DNA sequences are operably linked to gene transcription promoting elements;
(c) genetic engineering a cell line of interest, which comprises the genomic insertion, in the same cell, of a first expression vector of the DNA sequence encoding the reCG α-chain and a second expression vector bearing the DNA sequence which encodes reCG β-chain;
(d) independently expressing said first and second DNA sequences, in such a way that the α- and β-polypeptide chains are produced as separate molecules in the same cell of said cell line of interest, hence leading to the production of large amounts of the heterodimer formed between the α- and β-chains having eCG-like biological activity;
(e) isolating cell clones that produce reCG having *in vivo* biological activity;
(f) culturing the cell clones that produce reCG having *in vivo* biological activity; and
(g) recovering the reCG having *in vivo* biological activity from the culture medium conditioned by producing cell clones.

According to a second aspect of the present invention, there is provided a recombinant equine chorionic gonadotropin, produced from the improved process of the present invention, said reCG being produced by cDNA chains containing sequences that code for the α and β chains separately and wherein said reCG α and β polypeptide chains are fully or partially glycosylated.

In a third aspect, the present invention concerns a veterinary composition comprising:
(a) a veterinarily effective amount of the reCG produced in accordance with the present invention;
(b) optionally additives; and
(c) a pharmaceutically acceptable carrier.

In a fourth aspect, the present invention refers to the use of the reCG of the invention or the composition of the invention, comprising said reCG, in the manufacture of a medicament for the treatment of medical conditions related to the reproduction and ovulation of mammalian animals, as an optimizing agent of fertilization *in vitro;* in the manufacture of kits for diagnosing veterinary conditions and protocols related to mammalian ovulation; and in the manufacture of cell culture supplements.

### Brief description of the figures

Figure 1 illustrates the reCG α chain coding sequence along with its corresponding polypeptide sequence.
Figure 2 illustrates the reCG β chain coding sequence along with its corresponding polypeptide sequence.
Figure 3 illustrates the reCG α chain expression vector
Figure 4 illustrates the reCG β chain expression vector
Figure 5 illustrates the reCG yield after a 96h culture of the original mixed CHO-DG44 cGMP pcDNA 3.3-βeCG + pNU1-αeCG population and the enriched populations derived from this strategy. CHO-EYFP (negative control) is the medium conditioned for 96h by CHO-DG44 cGMP cells transfected with the pNU1 vector containing fluorescent protein EYFP coding message. The normalized reCG concentration in the assessed culture media is expressed in IU/mL per million viable cells.
Figure 6 illustrates the reCG yield, determined by specific ELISA, in media conditioned by the enriched cell populations generated during the second selection step with 30nM MTX and 750µg/ml Geneticin (G418). The normalized reCG concentration in the assessed culture media is expressed in IU/mL per million viable cells.
Figure 7 illustrates the reCG yield after 96h of cultivation of clones isolated by limiting dilution from the enriched CHO-DG44 cGMP E9 30/750 (pcDNA3.3-βeCG + pNU1-aeCG) and CHO-DG44 cGMP F9 30/750 (pcDNA3.3-βeCG + pNU1-aeCG) populations. The normalized reCG concentration in the assessed culture media is expressed in IU/mL per million viable cells.
Figure 8 illustrates the structural characterization of the reCGa+β heterodimer using *Western Blot.*
Figure 9 illustrates the typical electrophoretic profile obtained from purified reCGa+β observed after electrophoresis fractionation on a 12% non-denaturing polyacrylamide gel followed by silver staining.
Figure 10 illustrates the glycosylation profile obtained for the recombinant protein expressed by clone D3 (pcDNA3.3-βeCG + pNU1-aeCG).
Figures 11, 12 and 13 illustrate the *in vivo* functional evaluation of reCGa+β in rats by obtaining the ovarian and uterine response profiles (organ weight) after stimulation with different doses of reCGa+β.
Figure 14 illustrates the *in vivo* functional evaluation of reCGa+β in cows by obtaining the ovarian response profile (follicle overstimulation rate) after stimulation with different doses of reCGa+β.

### Detailed description of the invention

The present invention refers to an improved process for the production of a recombinant equine chorionic gonadotropin (reCG), wherein the α and β polypeptide chains are produced in the same cell and are fully or partially glycosylated. Said recombinant equine chorionic gonadotropin has a glycosylation profile similar to that of natural equine chorionic gonadotropin and exhibits in *vivo* FSH- and LH-hormone activity.

According to a first aspect of the invention, said improved process comprises the steps of:
(a) preparing and obtaining DNA sequences encoding reCG α and β chains;
(b) inserting said coding DNA sequences into expression vectors such that said coding DNA sequences are operably linked to gene transcription promoting elements;
(c) genetic engineering a cell line of interest, which comprises the genomic insertion, in the same cell, of a first expression vector of the DNA sequence encoding the reCG α-chain and a second expression vector bearing the DNA sequence which encodes reCG β-chain;
(d) independently expressing said first and second DNA sequences, in such a way that the α- and β-poiypeptide chains are produced as separate molecules in the same cell of said cell line of interest, hence leading to the production of large amounts of the heterodimer formed between the α- and β-chains having eCG-like biological activity;
(e) isolating cell clones that produce reCG having *in vivo* biological activity;
(f) culturing the cell clones that produce reCG having *in vivo* biological activity; and
(g) recovering the reCG having *in vivo* biological activity from the culture medium conditioned by the producing clones.

The *primers* used for amplification of the cDNA sequences encoding the eCG α- and β-chains were designed from the full-length coding sequences of each eCG subunit included in the NCBI database http://www.ncbi.nlm.nih.gov; *National Center for Biotechnology Information,* U. S. NLM - U. S. National Library of Medicine, Bethesda, USA (NM_access number α: 001099763.1, β: 001490342.2). No computer software was used to design the oligonucleotides.

Amplification of the coding chains can be carried out by RT-PCR (Reverse Transcription-Polymerase Chain Reaction) and the obtained products are separated and purified by techniques usually known and used in the state of the art. Preferably, the obtained amplicons are detected by agarose gel electrophoresis, isolated and purified using *lllustra GFX PCR DNA and Gel Band Purification Kit* (GE Healtcare, Carlsbad, CA, USA).

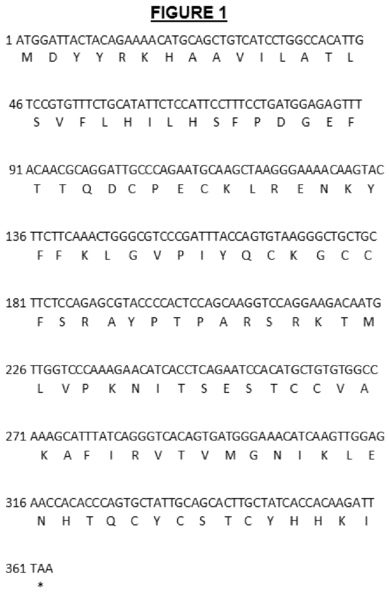

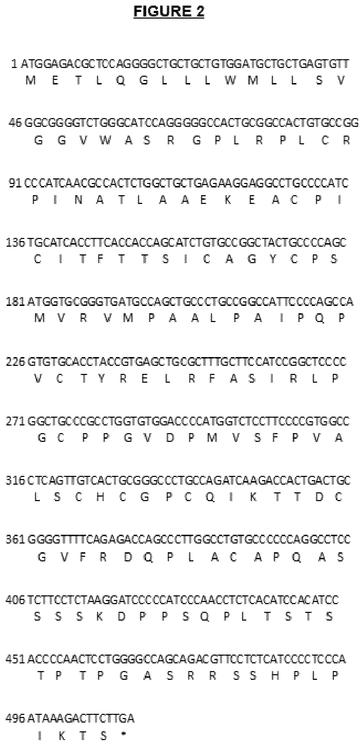

In another aspect of the invention, eCG α- or β-subunit coding sequences (SEQ ID NO. 1 and SEQ ID NO. 2, respectively) were subcloned into pNU1 and pcDNA 3.3 vectors, respectively. For sequencing the coding regions of aeCG and βeCG chains, pNU1-aeCG and pcDNA3.3-βeCG vectors were used, respectively, and primers annealing along the pcDNA 3.3 and pNU-1 expression vectors as well as the aeCG and βeCG inserts were used. The generated chromatograms and the DNASTAR package SeqMan software enabled the assembly of the consensus sequences from the individual sequences obtained. Consensus sequences were analyzed using BLASTn software (http://blast.ncbi.nlm.nih.gov/Blast.cgi, Basic Local Alignment Search Tool^{®}, NCBI, U. S. NLM), to verify similarity of these sequences with *Equus caballus* aeCG and βeCG consensus sequences, deposited at the NCBI database (NM_001197093.1). The consensus aeCG and βeCG chain coding nucleotide sequences are described as SEQ ID NO. 1 and SEQ ID NO. 2, respectively. The aeCG and βeCG chain nucleotide and polypeptide sequences can be seen in figures 1 and 2, respectively.
SEQ ID NO.1 
ATGGATTACTACAGAAAACATGCAGCTGTCATCCTGGCCACATTGTCCGTGTTTCT
GCATATTCTCCATTCCTTTCCTGATGGAGAGTTTACAACGCAGGATTGCCCAGAAT
GCAAGCTAAGGGAAAACAAGTACTTCTTCAAACTGGGCGTCCCGATTTACCAGTG
TAAGGGCTGCTGCTTCTCCAGAGCGTACCCCACTCCAGCAAGGTCCAGGAAGAC
AATGTTGGTCCCAAAGAACATCACCTCAGAATCCACATGCTGTGTGGCCAAAGCA
TTTATCAGGGTCACAGTGATGGGAAACATCAAGTTGGAGAACCACACCCAGTGCT
ATTGCAGCACTTGCTATCACCACAAGATTTAAAGGTTTCACCAAGTG
SEQ ID NO.2

ATGGAGACGCTCCAGGGGCTGCTGCTGTGGATGCTGCTGAGTGTTGGCGGGGTC
TGGGCATCCAGGGGGCCACTGCGGCCACTGTGCCGGCCCATCAACGCCACTCTG
GCTGCTGAGAAGGAGGCCTGCCCCATCTGCATCACCTTCACCACCAGCATCTGTG
CCGGCTACTGCCCCAGCATGGTGCGGGTGATGCCAGCTGCCCTGCCGGCCATTC
CCCAGCCAGTGTGCACCTACCGTGAGCTGCGCTTTGCTTCCATCCGGCTCCCCG
GCTGCCCGCCTGGTGTGGACCCCATGGTCTCCTTCCCCGTGGCCCTCAGTTGTC
ACTGCGGGCCCTGCCAGATCAAGACCACTGACTGCGGGTTTTCAGAGACCAGC
CCTTGGCCTGTGCCCCCCAGGCCTCCTCTTCCTCTAAGGATCCCCCATCCCAACC
TCTCACATCCACATCCACCCCAACTCCTGGGGCCAGCAGACGTTCCTCTCATCCC
CTCCCAATAAAGACTTCTTGA

The aeCG and βeCG chain polypeptide sequences are described as SEQ ID NO: 3 and SEQ ID NO: 4, respectively.
SEQ ID NO: 3: 
MDYYRKHAAVILATLSVFLHILHSFPDGEFTTQDCPECKLRENKYFFKLGVPIYQCKGC
CFSRAYPTPARSRKTMLVPKNITSESTCCVAKAFIRVTVMGNIKLENHTQCYCSTCYH
HKI
SEQ ID NO: 4:

METLQGLLLWMLLSVGGVWASRGPLRPLCRPINATLAAEKEACPICITFTTSICAGYCP
SMVRVMPAALPAIPQPVCTYRELRFASIRLPGCPPGVDPMVSFPVALSCHCGPCQIKT
TDCGVFRDQPLACAPQASSSSKDPPSQPLTSTSTPTPGASRRSSHPLPIKTS

The obtained aeCG and βeCG chain coding regions can be cloned into any plasmid vector containing one or more strong promoters, including chicken β-actin promoter and cytomegalovirus promoter, bacterial multiple cloning site (MCS), an antibiotic resistance sequence for bacterial cloning, recombination sites, and a cDNA encoding the enzyme dihydrofolate reductase (DHFR) downstream of an internal ribosome entry site (IRES). Preferably, the plasmid expression vector suitable for the invention is pNU-1 vector, which was designed by the applicant itself. However, other expression vectors containing similar functional elements can be used, such as, for example, pcDNA 3.3 vector, or other pcDNA family vectors.

pNU1 vector derives from the pUC18 vector (MESSING & VIEIRA, The pUC plasmids, an M13mp7-derived system for insertion mutagenesis and sequencing with synthetic universal primers. Gene. 1982 Oct;19(3):259-68), and has a MCS cloning cassette containing several restriction enzyme sites, the DNA coding sequences inserted into the MCS being operably linked and under the control of a hybrid promoter containing a cytomegalovirus enhancer element and a chicken β-actin promoter, as well as a cDNA that codes for dihydrofolate reductase (DHFR) enzyme downstream of an internal ribosome entry site.

This structure allows the concomitant expression of the cloned insert into the MCS, in this case aeCG or βeCG, and of DHFR, aiming at gene complementation of DHFR gene-double negative CHO cells, which, in turn, allows the selection of cells expressing high amounts of the product of interest after gene amplification performed through the treatment with increasing doses of DHFR inhibitors.

In a particular embodiment of the present invention, stable co-transfection of pcDNA3.3-βeCG and pNU1-aeCG vectors was carried out in CHO-DG44 cells to generate cell clones stably expressing the heterodimeric protein reCGa+β.

Insertion of plasmid expression vectors into the cell of interest (co-transfection) can be carried out by several techniques usually employed in the state of the art. Among them are several types of transfection, such as through cationic molecules, calcium-mediated transfection, PEI, electroporation and nucleofection.

In order to obtain the reCGa+β heterodimer, different alternatives can be used for the co-expression of aeCG and βeCG subunits. Thus, in an illustrative and non-exhausting manner, different expression vectors can be used to co-express different subunits of the reCG protein, such as, for example, the pcDNA3.3 expression vector to express the βeCG subunit, and the pNU1 expression vector to express the aeCG subunit. In this particular example, the pNU1 and pcDNA3.3 vectors have different selection markers for stable cell transfectants, namely: DHFR, in the case of pNU1 vector, and Neomycin Phosphotransferase II (NPTII) in the case of pcDNA3.3 vector, which facilitates the process of selecting stable positive co-transfectants, hence producing both subunits simultaneously. Stable co-transfection of pcDNA3.3-βeCG and pNU1-aeCG vectors can be performed using different molar ratios of the respective vectors, such as 2:1, or even 1:2, or preferably 1:1.

Since eCG is a complex molecule having around 45% of its molecular mass consisting of post-translational modifications (glycosylation), the use a robust expression system that allows the production of a recombinant eCG very similar to the wild-type eCG is required. The heterologous reCG expression system according to the present invention was chosen as it allows the correct processing of newly synthesized polypeptide chains, folding and assembly of their α and β subunits, in addition to adding complex-type glycan chains, which is essential for the produced recombinant eCG to exhibit biological activity. However, there is a lot of variability in the glycosylation patterns between different mammalian cells, and even within the same mammalian cell line, due to the fact that the enzymatic machinery of adding oligosaccharides to proteins differs between cell lines or between sub-phenotypes of a same cell lineage, generating glycoforms having different degrees and types of glycosylation and different biological activities. Due to the great variability of possible cellular sub-phenotypes relative to the glycosylation biosynthetic pathway composition allied to complexity of the glycosylation profile due to micro- and macro-heterogeneity phenomena, the use of an efficient strategy for phenotypic selection is required.

A particular embodiment of the present invention involves a method for identifying cellular sub-phenotypes by producing a reCG molecule having *in vivo* biological activity from an initial mixed cell population generated through stable co-transfection of reCG α- and β-chain expression vectors. The method provides a direct link between stable insertion events of reCG α- and β- chain expression vectors into the host cell genome and the production of biologically active forms of reCG without the need for manipulation or prior knowledge of the repertoire or gene expression profile of particular enzymes of the glycosylation biosynthetic pathway.

In a particular aspect of this method, according to the present invention, the eCG-like biological response is recognized by observing the induction of rapid and vigorous growth of uterine and ovarian tissues in immature rats. Thus, samples of medium conditioned by cell clones representing different cell sub-phenotypes are used to determine the eCG-like *in vivo* biological activity, hence identifying clones that produce biologically active forms of reCG.

The cells used to express reCG are mammalian cells or insect cells, or any cell line that is capable of providing gene expression profiles of the enzymes of the glycosylation biosynthetic pathway compatible with obtaining biologically active forms of reCG, even though the exact configuration of such expression profiles is unknown. Therefore, any other cell line that is capable of adding post-translational modifications similar to those obtained for the reCG profile as defined in the present invention, and having the same behavior when cultured *in vitro,* such as for example, replication capacity, protein expression capacity, simple transfectability, among others, can be used.

Preferably, mammalian cells were chosen; more preferably, CHO cell lines, or cells derived therefrom. Even more preferably, CHO-DG44-DHFR^{-/-} line is used. However, other cell types subject to the same phenotypic selection process, such as CHO, BHK, 293, Vero cells or derivatives thereof, are suitable for the invention and can replace the CHO-DG44-DHFR^{-/-} cells.

For the stable co-expression aeCG and βeCG subunits in CHO-DG44 cells, the initial step of selection of stable cell transfectants can be performed, for example, using nucleoside-free culture medium supplemented with the antibiotic G418, at a concentration range of from 200 µg/mL to 1 mg/mL, more preferably 500 µg/mL. Thereafter, the starting mixed cell populations can be subjected to additional selection cycles, after or without prior selection of cell subpopulations expressing higher levels of reCGa+β, by using increasing concentrations of DHFR enzyme inhibitors in the range of from 5 nM to 100 µM and the antibiotic G418 at a concentration range of from 500 µg/mL to 2mg/mL.

Various DHFR inhibitors are known in the art and may be suitable for use in the invention. Preferably, the DHFR inhibitor suitable for the invention is methotrexate (MTX).

Cells deficient in the production of DHFR enzyme, such as CHO-DG44 DHFR-/- cells, preferably used in the present invention, are suitable as they cannot survive when cultured in the absence of nucleosides, as this enzyme catalyzes the reduction of dihydrofolate to tetrahydrofolate. Thus, when these cells are transfected with constructs obtained from the vectors of the invention, which have the gene sequence corresponding to the DHFR enzyme, they become capable of growing in the absence of nucleosides.

reCG overproducing cells can be grown in monolayer or suspension production batches. Different reservoirs can be used, such as culture flasks of different sizes, plates, T-flasks, *spinners,* disposable culture bags (*bags*) and other types of bioreactors having rounded propellers or blades, hollow fiber, among other configurations.

The culture media used can vary as long as they are able to meet the metabolic needs of cells. Furthermore, reCG overproducing cells can be cultured in the presence or absence of fetal calf serum (FCS). The amount fetal calf serum can be adapted according to the culture conditions to maintain cell viability and protein expression levels (from 1 to 100 µg/mL) within the same order of magnitude as the original standard.

Chemically defined culture media or serum- and protein-free culture media, containing FCS substitutes, such as peptide growth and cell proliferation factors of various types, albumin, insulin, among others; or fetal or adult serum from other species, and being supplemented with precursors used in glycosylation pathways, are also suitable for use in the production of reCG. Cultivation must take place under defined environmental conditions, such as cultivation temperature between 25 and 40°C, relative CO₂ ratio between 1 and 10%, and pH between 5.0 and 8.0. Preferably, for the production of the reCGa+β heterodimer, the producing cell clones are cultured in suspension and in a chemically defined culture medium. When cultivation is carried out in spinners, bags and bioreactors, the rotation used can vary between 10 and 200 rpm.

To achieve greater mass and purity of the protein of interest, the obtained product can be partially purified by using known techniques for protein purification, wherein the reCGa+β-containing conditioned culture media are subjected to at least one of the following techniques: protein precipitation , for example, in the presence of ammonium sulphate; ultrafiltration; tangential filtration; or two distinct types of chromatography, that is, ion exchange chromatography and affinity chromatography.

According to a second aspect of the present invention, there is provided a recombinant equine chorionic gonadotropin produced from the process of the present invention. Unlike commercially available options, the recombinant equine chorionic gonadotropin of the invention is produced through biotechnological mechanisms on an industrial scale, offering an alternative option to those obtained from equine plasma and involving bioethical factors. In addition, the reCG of the invention will not face the issues found in the state of the art related to the scarcity of the source of origin, advantageously constituting a new source of uninterrupted supply of reCG.

By producing a reCG with a glycosylation pattern similar to that of commercially available chorionic gonadotropins and having the same level of *in vivo* activity as both the FSH and LH hormones, a product with at least similar biological activity is achieved. This feature is also absent in the recombinant chorionic gonadotropins known in the state of the art, which did not even exhibit biological activity *in vivo.*

In a third aspect, the present invention concerns a veterinary composition comprising:
a) a veterinarily effective amount of the reCG produced in accordance with the present invention;
b) optionally additives; and
c) a pharmaceutically acceptable carrier.

The composition of the present invention may comprise, in addition to the reCG of the invention, adjuvants, preservatives, diluents, emulsifiers, stabilizers and other pharmacologically acceptable ingredients which are employed in gonadotropin preparations for animal use.

In a fourth aspect, the present invention refers to the use of (a) the reCG or (b) the veterinary composition of the invention, comprising said reCG, in the manufacture of a medicament for the treatment of veterinary conditions related to the reproduction and ovulation of mammalian animals, as an optimizing agent of *in vitro* fertilization; in the manufacture of kits for diagnosing veterinary conditions and protocols related to mammalian ovulation; and in the manufacture of cell culture supplements.

### EXAMPLES

### Example 1: RNA extraction and cDNA synthesis from equine pituitary

A pituitary gland was obtained in the autopsy of a 19-year-old, male, partbred arabian horse (*Equus caballus*), and immediately packaged in a *RNAholder* (Bioagency^{©} Biotechnology) at 4 ° C until being macerated in liquid nitrogen using a crucible and a pistil. Total RNA was extracted from this pituitary macerate using *Illustra RNAspin Mini RNA Isolation Kit* (GE Healthcare^{®}, Buckinghamshire, England), following the manufacturer's recommendations. The obtained RNA concentration and purity were determined by quantification in a nanospectrophotometer (ND-1000 *Spectrophotometer,* NanoDrop Technologies Inc., Wilmington, USA) and the absorbance ratio Abs260/Abs280 and Abs260/Abs230 nm was read.

A 1.0µg aliquot of total equine pituitary RNA was used as a template for reverse transcription reaction using the *lmProm^{™}-ll RT* enzyme kit (Promega Corporation, Madison, USA); *Super Script II* (Invitrogen), with reactions being performed according to the manufacturer's recommendations. At the end, the samples were diluted 3 times in deionized water obtaining a final volume of 60µL and stored at -80°C.

### Example 2: Amplification of eCG α and β coding chains from this cDNA library by PCR

*Primers* used for amplification of the cDNA sequences were designed based on the full-length coding sequences of each of the two eCG subunits present in the NCBI database (http://www.ncbi.nlm.nih.gov; *National Center for Biotechnology Information,* U. S. NLM - U. S. National Library of Medicine, Bethesda, EUA (NM_access number α: 001099763.1, β: 001490342.2) and synthesized by Thermo Fisher Scientific Inc., Waltham, USA ^{™}/Thermo Fisher Scientific Inc. The lyophilized *primers* were reconstituted in 10mM Tris-CI buffer solution (pH 8.0) to generate a 100µM stock solution and 10µM use solutions. The *primer* sequences used to amplify these chains and their intermediates obtained in Genetic Engineering steps are listed in Table 1.

**Table 1. Primer sequences for amplification of reCGα and reCGβ coding sequences.**

| | |
|---|---|
| **Oligonucleotides** | **Sequences (5'-3')** |
| 1 - eCGb F | 5'-ATGGAGACGCTCCAGGGGCT- 3' |
| 2 - eCGb R | 5'-AGAAGTCTTTATTGGGAGGGGATGAGAG- 3' |
| 3 - eCGa F | 5'-TTTCCTGATGGAGAGTTTACAACGCAG- 3' |
| 4 - eCGa R | 5'-TTAAATCTTGTGGTGATAGCACGTGCTG-3'. |
| 5 - Mut eCGa R *Not*l | 5'-GC**GCGGCCGC**TTAAATCTTGTTGTGGTG-3' |
| 6 - Mut eCGb F EcoRI | 5'-CG**GAATTC**GCCACCATGGAGACGCTC-3' |
| 7 - union F | 5'-CCCAATAAAGACTTCTTTTCCTGATGGAGAG-3' |
| 8 - union R | 5'- CTCTCCATCAGGAAAAGAAGTCTTTATTGGG-3' |
| 9 -Mut EcoRI eCGa F pep | 5'GCG**GAATTC**GCCACCATGGATTACTACAGAAAACATGC3' |
| 10- Mut Notl eCGb R stop | 5'CGC**GCGGCCGC**TCAAGAAGTCTTTATTGGGAG3' |

Bolded letters: *Eco*RI *(forward primer)* and *Not*I (*reverse primer*) enzyme restriction sites; underlined: Kozak consensus sequence.

Total cDNA from equine pituitary tissue was used for amplification of the coding sequences for both eCG subunits. *Primers* (9) and (5) were used to amplify the full-length eCGβ coding sequence, and the full-length eCG coding sequenceα was amplified using *primers* (6) and (10).

For amplification, polymerase chain reactions (PCR) were performed using *High Fidelity Platinum Taq DNA Polymerase* enzyme (Thermo Fisher Scientific Inc., Waltham, USA^{™}). To 100 ng of equine pituitary cDNA samples were added 1X *High Fidelity PCR Buffer* (Thermo Fisher Scientific Inc., Waltham, USA), 0.2 mM dNTPs (Thermo Fisher Scientific Inc., Waltham, USA), 2mM MgSO₄ (Thermo Fisher Scientific Inc., Waltham, USA), 400 nM *Forward Primer* (Thermo Fisher Scientific Inc., Waltham, USA), 400 nM *Reverse Primer* (Thermo Fisher Scientific Inc., Waltham, USA), 1.0 U of the above DNA polymerase enzyme and deionized water q.s. 50.0 µL of reaction volume. The reaction was carried out under the following conditions: 94°C for 1 minute; 35 cycles of: 94°C for 30 seconds, 55°C (eCGα ) or 58°C (eCGβ) for 30 seconds and 68°C for 1 min; final extension at 68°C for 2 min.

Amplicons were detected using 2.0% agarose gels stained with 0.5µ g/mL ethidium bromide, and the expected size product was isolated with a scalpel and purified using *GFX DNA and Band Purification Kit* (GE Healtcare, Carlsbad, CA, USA), according to the manufacturer's instructions.

### Example 3: Cloning of eCGa and eCGβ chains into pcDNA 3.3 and pNU-1 expression vectors for mammalian cells

To obtain the reCG alpha chain expression vector, pNU1-αeCG, the purified DNA fragment, previously obtained from a PCR reaction with *primers* 5 and 9 as described in Table 1 and containing the region encoding aeCG was digested with *EcoR* I and *Not* I restriction enzymes, followed by thermal inactivation of the enzymes. Ligation of the purified aeCG DNA fragment and the pNU1 vector co-digested with *EcoR* I and *Not* I restriction enzymes was performed. Aliquots of the binding reactions were used to transform *Escherichia coli* DH10B strain bacteria by electroporation. For selecting recombinant colonies containing the pNU1-aeCG vector, 100µ g/mL ampicillin was used. Obtainment of the recombinant plasmid was confirmed by obtaining its restriction profile with *EcoR* I and *Not* I enzymes. Construction of pcDNA3.3-βeCG vector was performed by subcloning the βeCG chain originally present in pNU1-β eCG plasmid. To this end, vector pNU1-βeCG was digested with *Xba* I and *Mss* I restriction enzymes to release the βeCG coding region and its purification by agarose gel electrophoresis and silica adsorption. Ligation of the purified βeCG DNA fragment and the pcDNA3.3 vector co-digested with *Nhe* I and *Mss* I restriction enzymes was performed. Aliquots of the binding reactions were used to transform *Escherichia coli* XL1-Blue strain bacteria by electroporation. For selecting recombinant colonies containing the pcDNA3.3-βeCG, vector, 100µ g/mL ampicillin was used. Obtainment of the recombinant plasmid was confirmed by obtaining its restriction profile with *Mlu*l enzyme.

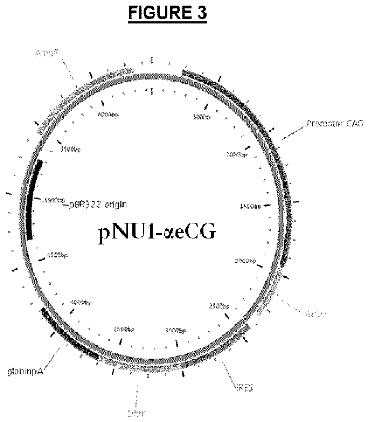

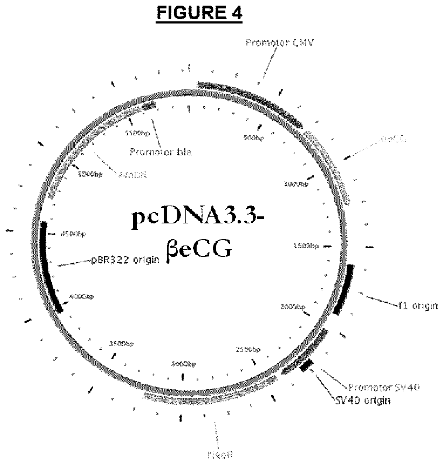

pNU1-aeCG and pcDNA3.3-βeCG vectors were characterized by automated DNA sequencing using Big Dye^{®} kit (Thermo Fisher Scientific^{™}) and ABI PRISM 3700 DNA Analyzer (Thermo Fisher Scientific^{™}) and various primers that anneal along the pNU1 and pcDNA3.3 expression vectors, and to aeCG and βeCG inserts. Assembly of the consensus sequences of vectors pNU1-aeCG and pcDNA3.3-βeCG was performed from the individual sequences obtained using DNASTAR package SeqMan software. The schematic maps of pNU1-aeCG and pcDNA3.3-βeCG expression vectors are shown in Figures 3 and 4, respectively.

### Example 4: Stable co-transfection by lipofection of pcDNA 3.3-βeCG and pNU1-αeCG constructs into CHO-DG44 cells grown in suspension and maintained in defined medium.

CHO-DG44 cGMP cells used for stable co-transfection of aeCG and βeCG chains are part of the Freedom DG44 system (Invitrogen ^{®} ). Cells were cultured according to the manufacturer's instructions in suspension and under stirring (100-120 rpm), in a "Spinner" type flask containing 30mL of CD DG44 medium supplemented with 8mM L-glutamine and 0.18% Pluronics, in a moist atmosphere at 37°C and 8% CO₂. At every 3 or 4 days the cell culture was assessed for cell density and percentage of viable cells, and subcultured to a density of about 3×10⁵ viable cells/mL.

pNU1-aeCG and pcDNA3.3-βeCG vectors were linearized with *Pvu* I restriction enzyme to optimize the insertion process of the expression cassettes of interest into the CHO-DG44 cell genome. After linearization, plasmids were purified by precipitation and quantified by spectrophotometry. For co-transfection, 9 µg of each plasmid was mixed with 600µl of OptiPro SFM medium (Invitrogen). In parallel, 15 µl of *FreeStyle Max Reagent* (Invitrogen) was mixed with 600 µl of OptiPro SFM medium (Invitrogen). Then, the two mixtures were combined and incubated for 10min at room temperature to allow complexes to form. Subsequently, the mixture of DNA and FreeStyle reagent was added to the cells dropwise (1.15 × 10⁷ viable cells) and the suspension was gently shaken. Then, the culture was kept in an incubator at 37°C and 8% CO ₂.

### Example 5: Selection of stable transfectants from CHO-DG44 cells stably co-transfected with pcDNA 3.3-βeCG and pNU1-αeCG expression vectors

About 48h after co-transfection the cells were transferred to the selection medium (OptiCHO supplemented with 8mM glutamine and 500µg/ml G418). After about 20 days of cultivation in the selection medium, a selection medium-resistant cell subpopulation was achieved which, therefore, carries at least one copy of each of the vectors of interest integrated into its genome.

The 96h-conditioned medium of this original mixed population was collected to evaluate the yield of reCGa+β by a specific ELISA (*Equine Pregnant Mare Serum Gonadotropin ELISA-* DRG kit), following the manual provided by the manufacturer. Absorbance values were determined at 450nm by reading on a plate spectrophotometer (SpectraMax M2 - Molecular Devices). A standard curve provided by the kit was used as a reference for a 4-parameter logistic analysis. reCG concentration in the culture media was normalized to the number of viable cells at the time of collection. A yield of 1.3 Ul/mL/million viable cells was obtained in the initial mixed population.

### Example 6: Cell enrichment from mixed CHO-DG44 eCGa + eCGβ population based on reCG expression levels

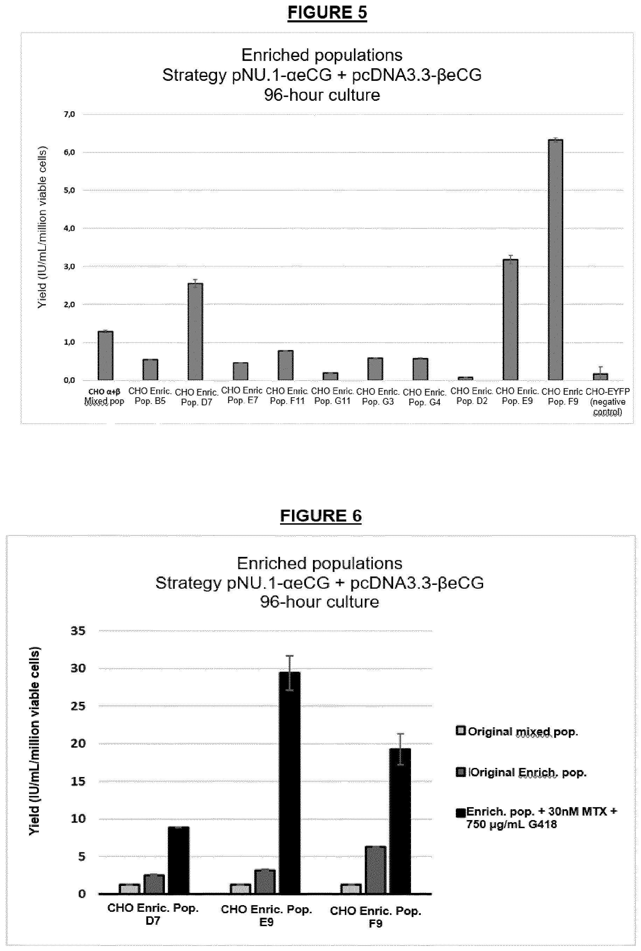

The enrichment protocol was performed by serial dilution of the mixed CHO-DG44 cGMP pNU1-aeCG + pcDNA3.3-βeCG population in culture medium (FortiCHO + 6mM L-glutamine + 1X HT supplement + 10% medium conditioned for 96 hours by the original mixed cell population) until obtaining a theoretical concentration of 2 viable cells per 200µL of medium. These cells were seeded into 96-well plate wells and grown in an incubator at 37°C and 5% CO₂ for up to 25 days. The described protocol generated 10 populations, which were expanded until cultures were obtained in a volume of 5mL. Media conditioned for 96h of these populations were collected and assessed for their yields by ELISA (*Equine Pregnant Mare Serum Gonadotropin ELISA*-DRG) to validate the obtained enrichment (Figure 5). The assay was performed with freshly collected, conditioned culture media diluted 100X in the kit diluent (Zero Standard). As a negative control, medium conditioned for 96 hours by CHO-DG44 cells stably transfected with pNU1-EYFP vector was used. reCG concentration in the culture media was normalized to the number of viable cells at the time of collection.

In Figure 5, it can be seen that the enrichment protocol of the mixed CHO DG44 cGMP pNU1-aeCG + pcDNA3.3-βeCG population provided three populations enriched in reCG-producing cells, namely: the D7, E9 and F9 enriched populations, which express about 2, 2.5 and 5 times more reCG, respectively, than the original mixed population.

### Example 7: Enrichment of cell subpopulations with higher reCG expression levels by selecting enriched CHO-DG44 eCGa + eCGβ E9 and F9 cell populations with increasing doses of MTX and G418

A second round of selection of enriched mixed CHO DG44 cGMP D7 (pNU1-αeCG + pcDNA3.3-βeCG), CHO DG44 cGMP E9 (pNU1-αeCG + pcDNA3.3-βeCG) and CHO DG44 cGMP F9 (pNU1-αeCG + pcDNA3.3-βeCG) populations was performed by adding to the culture medium (OptiCHO supplemented with 8mM Glutamine) 30nM MTX and 750µg/mL G418 until cell populations resistant to these drug doses were obtained, that is, populations having cell viability greater than 85%.

Assessment of yield of cell populations generated by the second-round protocol of MTX and G418 selection was performed using the anti-PMSG ELISA kit (*Equine Pregnant Mare Serum Gonadotropin ELISA* - *Alpco*), according to the manufacturer's instructions. As a negative control, medium conditioned for 96 hours by CHO-DG44 cells stably transfected with pNU1-EYFP vector was used. reCG concentration in the culture media was normalized to the number of viable cells at the time of collection. The results are shown in Figure 6.

In figure 6, the second round of MTX and G418 selection is shown to allow selection of cell subpopulations having yields greater than those of the original enriched populations. Among them, emphasis is given to mixed CHO DG44 cGMP E9 (pNU1-aeCG + pcDNA3.3-βeCG) 30nM MTX + 750µg/mL G418 (30/750) subpopulation, whose increase in yield was about 9x over the original enriched population. All other cell subpopulations showed increased yields of the order of 3x greater than the original enriched populations.

### Example 8: Isolation of cell clones having higher reCG expression levels from enriched CHO-DG44 eCGa + eCGβ E9 and F9 30 MTX/750 G418 cell populations

Isolation of cell clones was performed as recommended by the manufacturer in the Freedom^{™} system manual [Freedom^{™} DG44 Kit - For transfection of CHO DG44 Cells (cGMP banked) and development of stable cell lines for protein production - Publication Part Number MAN0003649 - Revision Date 27 April 2011, pp 53 to 57]. A serial dilution of cell cultures of the enriched CHO DG44 cGMP E9 30/750 (pNU1-αeCG + pcDNA3.3-βeCG) and CHO DG44 cGMP E9 30/750 (pNU1-αeCG + pcDNA3.3-βeCG) populations was performed in such a way that, at the end of this procedure, a cell suspension having a theoretical concentration of 37.5 viable cells/mL was obtained.

20µL of these cell suspensions were distributed per well of 384-well non-adherent culture plates. Thus, the cell density per well was theoretically 0.75 cell/well. The culture medium used during clone isolation consisted of FortiCHO, 6mM L-glutamine, 1X HT supplement, 1mM Sodium Pyruvate, and 10% medium conditioned for 96 hours by the respective mixed cell population. The 384-well plate was kept in an incubator at 37°C and 5% CO₂.

About 2 hours after plating, the number of cells per well was evaluated under a microscope and part of the wells containing cells was found to have a single cell. Cell growth was assessed every three or four days. Clones that reached a cell density in the range of hundreds of cells/well were initially transferred to wells of a 96-well non-adherent cell culture plate, later to wells of a 24-well non-adherent cell culture plate, and, finally, to non-adherent T25 flasks, under regular cultivation conditions, that is, OptiCHO medium supplemented with 8 mM glutamine, and kept at 37°C and 8% CO₂. Nine clones were obtained from the enriched CHO DG44 cGMP E9 30/750 (pNU1-aeCG + pcDNA3.3-βeCG) population and 10 clones were obtained from the enriched CHO DG44 cGMP F9 30/750 (pNU1-αeCG + pcDNA3.3-βeCG) population.

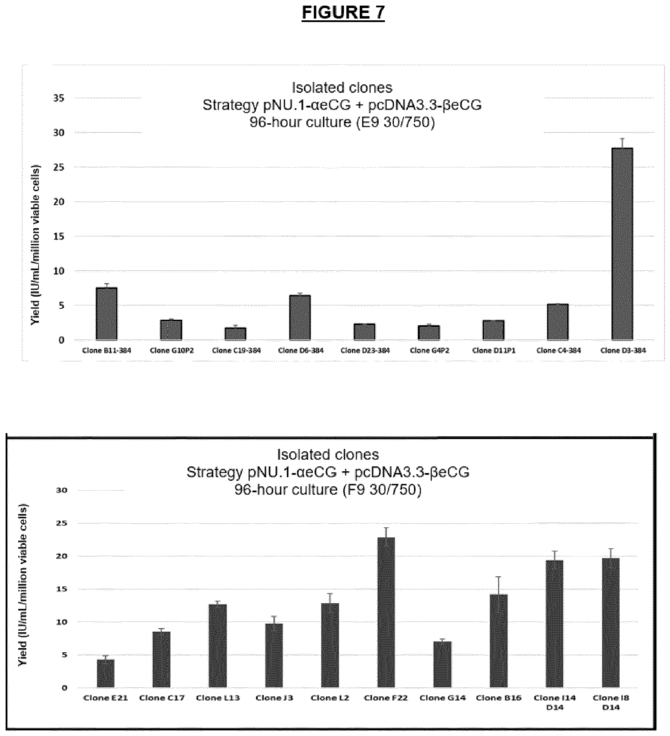

Yield of reCGa+β of the isolated clones was evaluated by eCG-specific ELISA (*Equine Pregnant Mare Serum Gonadotropin ELISA* - *Alpco*), according to the manufacturer's instructions. To carry out the assay, culture media conditioned for 96 hours and newly collected were used. As a negative control, medium conditioned for 96 hours by CHO-DG44 cells stably transfected with pNU1-EYFP vector was used. eCG concentration in the culture media was normalized to the number of viable cells at the time of collection. The results are shown in Figure 7. A clone having a yield of about 28 IU/mL/million viable cells was obtained (clone D3 pnapcb).

### Example 9: Structural characterization of reCGα+β by Western blot

Western blot was used for the specific structural characterization of recombinantly produced eCG molecules, both in their fused form reCGβa and the heterodimer reCGa+β. Samples of 96h conditioned medium were collected from the culture of CHO-DG44 cGMP βαeCG E11-300nM cells which produce the fused form, reCGβa, from CHO-DG44 cGMP a+β D3 pnapcb cells, which produce the heterodimer reCGa+β, and from CHO-DG44 cGMP EYFP cells (negative control). eCG quantification in the samples was performed by anti-PMSG ELISA method (Equine Pregnant Mare Serum Gonadotropin ELISA-DRG). Later, the samples and the positive control (Novormon) were concentrated by ultrafiltration (Filter Amicon Ultra 0.5 10K) to a final titer of 8000 IU/mL. Volume of conditioned medium of the negative control used was calculated so as to proportionally represent the number of viable cells present at the time of collection of CHO-DG44 cGMP βαeCG E11-300nM cells, and the volume used in the analysis was 75lU reCG. Thereafter, the calculated volume of negative control was concentrated by the same concentration factor used for sample E11-300nM. Three different amounts of eCG (25, 50 and 75 IU) were evaluated by electrophoresis (12% SDS-PAGE) under non-reducing conditions and without prior heat denaturation. After electrophoresis, proteins were transferred to a nitrocellulose membrane by wet transfer for two hours. After the transfer, the membrane was blocked with 5% skim milk solution in 0.05% TBS-Tween for one hour. Then, the membrane was labeled for one hour with primary anti-PMSG antibody (AP02036SU-N - Acris) diluted 1:2000 in blocking solution, and incubated at room temperature. Subsequently, three 10-min washes of the membrane were carried out with a 0.05% TBS-Tween solution, and, then the membrane was incubated for 45min with secondary anti-rabbit IgG antibody (HRP (horseradish peroxidase)-conjugated anti-rabbit antibody- Sigma-Aldrich #A4914) diluted in blocking solution at 1:2,000 dilution. After three 10min-washes with TBST, the membrane was incubated for 5min with the peroxidase enzyme substrate and the luminescent signal was recorded on a radiographic film.

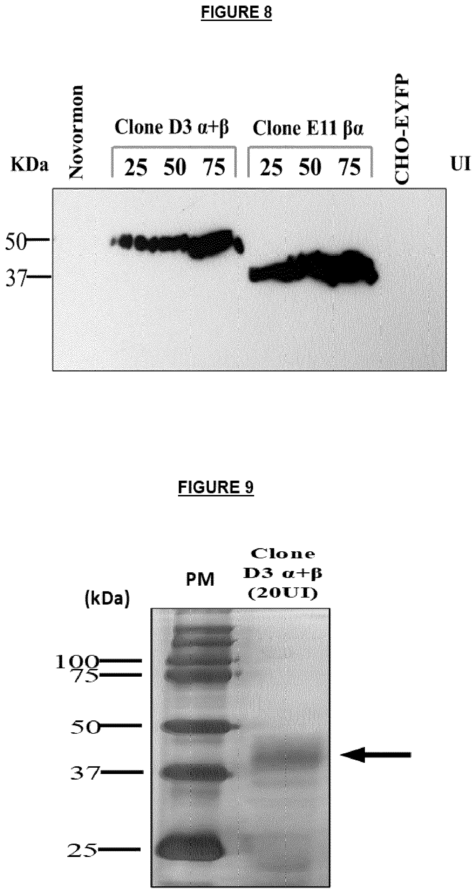

reCGβa produced by clone E11-300nM and reCGa+β produced by clone D3 pnapcb showed immunoreactivity against the anti-eCG antibody used (Figure 8). Additionally, reCGβa produced by clone E11-300nM was found to have an apparent molecular weight of approximately 40 kDa, and reCGa+β produced by clone D3 pnapcb was found to have an apparent molecular weight of 45 kDa under non-reducing conditions and without prior heat denaturation (Figure 8).

### Example 10: Purification of reCGα+β by cation exchange chromatography

Cation exchange chromatography was performed using OptiCHO culture medium conditioned for 96h by cell clone CHO-DG44 cGMP aeCG + βeCG D3 pnapcb. Before starting purification, pH of the conditioned medium was adjusted to 6.5 by adding 10% HCI, which was then filtered through a 0.45µm filter. Cation exchange chromatography was performed using Hitrap SP Fast Flow commercial column (90 µm particles, 16 × 25mm size, 5mL volume) with the aid of Akta Purifier UPC 100 chromatographic system and Unicorn 5.31 softaware (GE Healthcare, Uppsala, Sweden). Channel A1 was fed with 0.1M NaCl and 8mM sodium acetate pH 3.5 buffer, channel A2 with 0.1M Na₂HPO₄ pH 4.4 buffer, channel B1 with 0.8M NaCl and 8mM sodium acetate pH 3.5 buffer, and channel B2 with 2M NaCl and 20mM Tris pH 7 buffer. Throughout the chromatography procedure, a flow rate of 2 mL/min was used. The procedure was started by equilibrating the column with 15 mL OptiCHO medium with the pH adjusted to 6.6 using 10% v/v HCI. After sample injection, the first (buffer A1) and second (buffer A2) washing steps were carried out using 5 column volumes (CV) per wash. Elution was performed in an isocratic regimen, at a flow rate of 2 mL/min, using 5 CV of buffer in lane B1, and then washing and reconstitution of the column was made with 3 CV of buffer in lane B2. During the procedure, the fraction corresponding to the second 5 mL fraction referring to the elution step was collected. Figure 9 depicts the typical electrophoretic profile obtained from purified reCGa+β observed after electrophoresis fractionation on a 12% non-denaturing polyacrylamide gel followed by silver staining.

### Example 11: Analysis of reCG glycosylation profile

The methodology used for the qualitative assessment of the glycosylation profile present in reCGa+β was adapted relative to that described by Legardinier et al. (LEGARDINIER et al., Mammalian-like nonsialyl complex-type N-glycosylation of equine gonadotropins in Mimic insect cells. Glycobiology. 2005 15(8): 776-90), and which is based on the use of a panel of lectins to detect specific residues of oligosaccharide chains in the reCG molecule. Firstly, it involves immobilizing the purified proteins to be analyzed in chemically treated 96-well plate wells for protein adsorption optimization (NUNC-Immuno Plate/MaxiSorp-NUNC) and subsequent probing by lectins that specifically recognize different glycan structures.

About 200 µg of the purified proteins produced by clone D3 pnapcb were diluted in 0.1 M sodium carbonate buffer pH 9.6, and dispensed into wells of the aforementioned plate. Immobilization was performed in a wet chamber for about 16 hours. After this step, the wells were washed with phosphate saline solution free of Ca²⁺ and Mg²⁺ (PBSA), and blocked with TBS buffer + 0.05% Tween20 + 2% Polyvinylpyrrolidone K30 for 2 hours at 4°C.

After another wash, the step of binding the lectins to specific residues of the glycan side chains was carried out. A panel of five lectins conjugated to digoxigenin, which are components of a commercial kit (*DIG Glycan Differentiation Kit* - *Roche*) was used. The conjugated lectins used and their respective recognized glycan residues are summarized in Table 2 below:

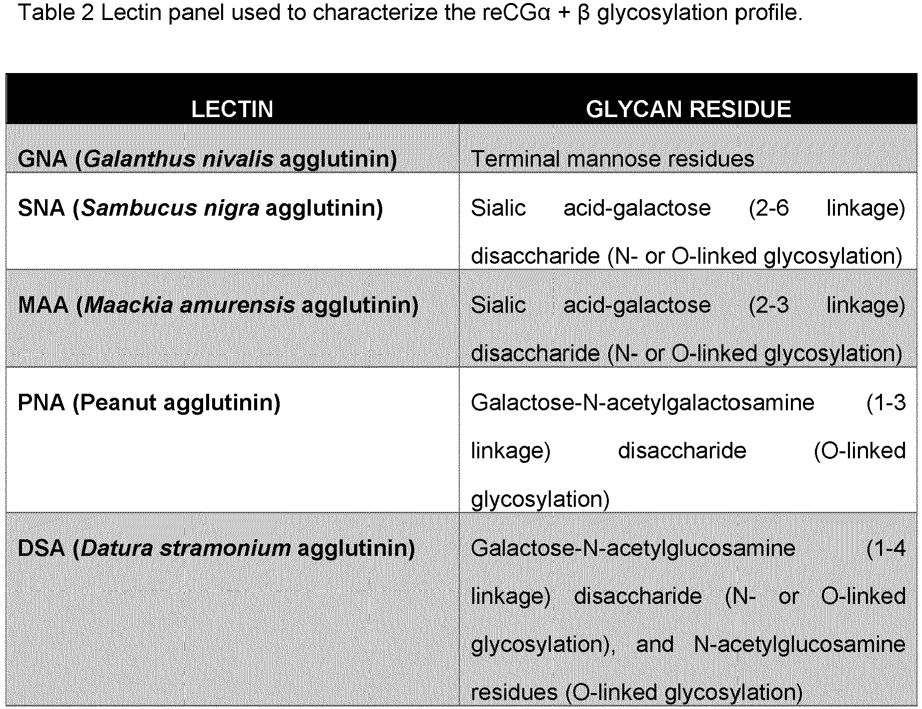

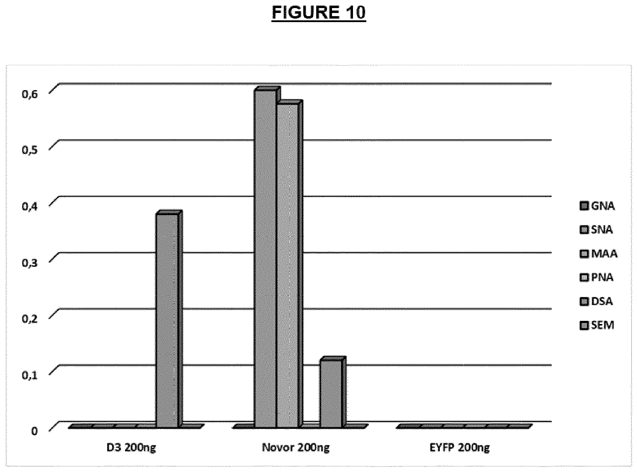

The lectin binding step is followed by a washing step, and, subsequently, the incubation step with an anti-digoxigenin antibody conjugated to alkaline phosphatase enzyme, an additional washing step, followed by the detection step using alkaline phosphatase enzyme substrate BCIP (5-bromo-4-chloro-3-indolyl phosphate). The reaction is quenched with EDTA and spectrophotometric reading is taken at 600nm. As a negative control, the medium conditioned for 96h by CHO-DG44 cells stably transfected with the pNU1-EYFP vector was used, which was subjected to the same purification protocol used for the purification of reCG, as described above. As a positive control, 200 ng of eCG (Novormon) was used. The assay was performed in duplicate for each lectin. The results are shown in Figure 10.

The pattern observed for Novormon positive control indicates that the wild-type eCG molecule has a glycosylation profile that includes terminal sialylation at the O- and N-linked residues (SNA and MAA lectins), compatible with that described in the literature (LEGARDINIER et al., Mammalian-like nonsialyl complex-type N-glycosylation of equine gonadotropins in Mimic insect cells. Glycobiology. 2005 15(8): 776-90). Regarding the reCGa+β produced by clone D3 pnapcb, an incomplete glycosylation pattern was observed, with no terminal sialylation at the O- and N-linked residues (DSA lectin), indicating termination of the glycan chain at the Gal-(1-4)GIcNAc disaccharide unit on N- and/or O-linked glycan chains, and/or termination of the glycan chain at the GlcNAc monosaccharide unit as the O-linked terminal residue.

### Example 12: Assessment of the in vivo biological activity of reCGα+β

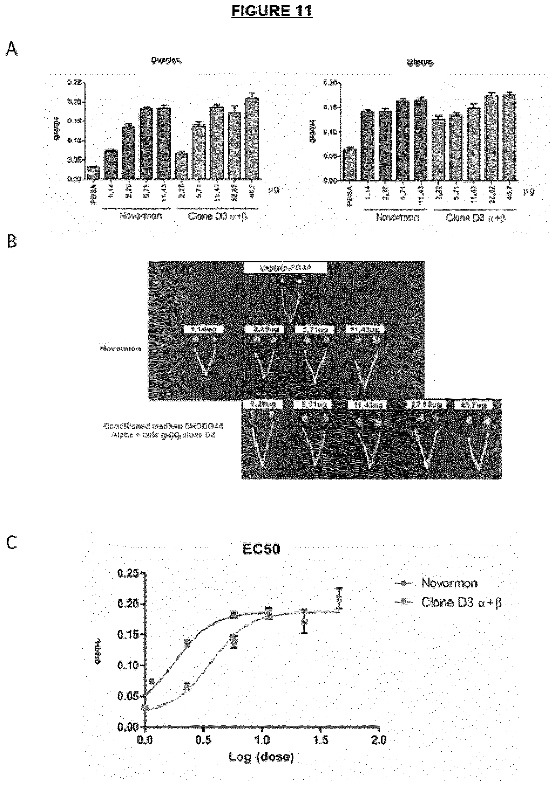

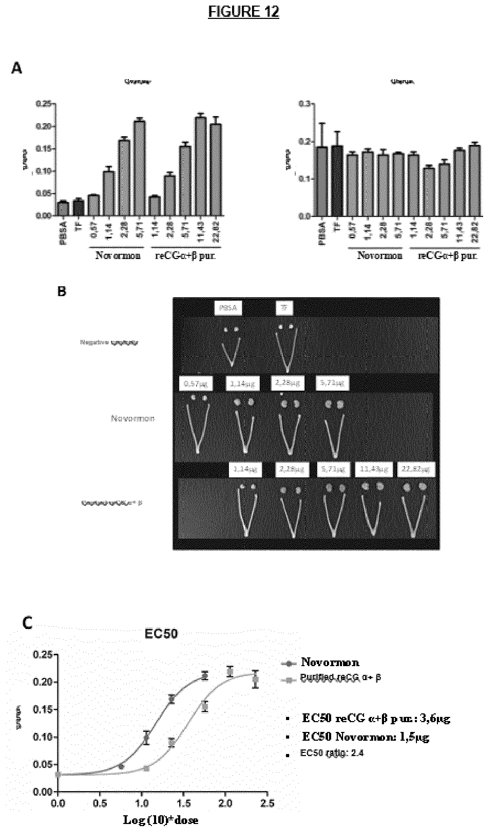

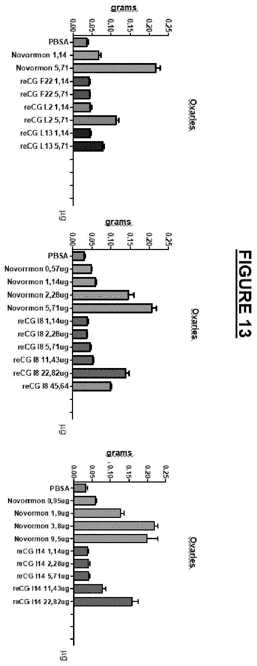

All experiments involving animals as well as methods adopted for breeding and forms of maintenance and experimentation followed the rules of the Ethics Committee on Animal Experiments (CEUA) of the USP Institute of Chemistry. The study was carried out using the British Pharmacopoeia-recommended assay as described by Cole & Erway (Cole & Erway, 1941). The animals were arranged into groups of 5 to 10 animals per box, receiving sterilized water and feed *ad libitum,* and kept for periods of 12 hours of light and 12 hours of darkness at a constant temperature of 22°C. The assay for evaluating the *in vivo* biological activity of eCG is based on the determination of the dose-response effect of the hormone on the ovarian and uterine weights of prepubertal rats. For the *in vivo* functional evaluation of recombinant reCGa+β produced by cell clone D3 pnapcb, both samples of culture medium conditioned for 96h by these cells (Figure 11) and a preparation of reCGa+β purified by cation exchange chromatography from a sample of medium by conditioned for 96 hours by clone D3 pnapcb (Figure 12) were used. Also, the *in vivo* biological activity of reCG present in media samples conditioned for 96h by additional producing clones (clones F22, L2, L13, I8D14 and I14D14) (Figure 13) was evaluated. The conditioned medium samples were concentrated by ultrafiltration using Vivaspin 20MI and 10kDa cutoff columns (GE Healthcare). After the concentration step, the amount of reCGa+β was estimated by ELISA in the concentrated conditioned medium samples and in the purified preparation of reCGa+β, and subcutaneous injection of reCGa+β was carried out in Sprague-Dawley rats aged between 23 and 25 days. To obtain the dose-response curve, reCGa+β doses of 1.14, 2.28, 5.71, 11.4, 22.8 and 45.7 µ g diluted in PBSA (diluent) were used. For the commercial reference product, eCG obtained from the plasma of pregnant mares (Novormon), doses of 1.14, 2.28, 5.71 and 11.4 µ g diluted in PBSA were used. As a negative control, vehicle was injected. Injections were performed in 5 animals per dose or experimental group, and approximately 72 hours after injection, the animals were euthanized in a CO₂ chamber, and their ovaries and uterus were dissected, dried and weighed. The final response for each dose was obtained through the arithmetic mean of the weight of organs (ovaries or uterus) of the five animals at each point. An increased weight of these organs compared to negative controls corresponds to the animal's biological response to the eCG stimulus. Regarding clone D3 pnapcb, figures 11A (conditioned medium samples) and 12A (purified reCG samples) represent the profiles of ovarian and uterine responses to stimulation with the different reCGa+β doses assessed, figures 11B (conditioned medium samples) and 12B (purified reCG samples) represent the morphological aspects of ovaries and uterus under different experimental conditions explored, and figures 11C (conditioned medium samples) and 12C (purified reCG samples) represent the dose-response curves obtained for the ovaries, which were obtained by 4-parameter logistic regression made by *GraphPad Prisma 5.00 for Windows* software (GraphPad Software, San Diego California USA, www.graphpad.com), and used to calculate the effective dose (ED-50 or ED-50) for reCGa+β (clone D3 pnapcb) and Novormon. Treatment with reCGa+β (clone D3 pnapcb) was shown to present an uterine and ovarian response profile very similar to that observed after treatment with the reference product, with an ED50 ratio of both hormones of approximately 2. However, the same pattern of ovarian responses was not observed in samples of medium conditioned by other clones (Figure 13, clones F22, L2, L13, I8D14 and I14D14), in one of them (clone F22), despite exhibiting a volumetric reCG yield (-20 IU/10⁶cells/96 hours) similar to that of other clones, the reCG produced by said clone did not show any biological activity *in vivo.* These results demonstrate the heterogeneity of the reCG forms produced by different cell clones, and how the use of the clonal scanning tool based on the observation of the induction of rapid and vigorous growth of uterine and ovarian tissues in immature rats is essential to identify arrangements of the reCG molecule having *in vivo* biological activity.

### Example 13: Clinical trial carried out in anestrus cows

A clinical trial was carried out in anestrus cows with the reCGa+β product produced by clone D3 pnapcb. The study involved 40 Nellore cows (*Bos taurus indicus*) and/or crossbred cows (*Bos taurus taurus X Bos taurus indicus*) aged between 16 months and 10 years, having a weight compatible with the category and body condition score (BCS) ≥ 2.5 (scale from 1 to 5). The animals were kept on a *Brachiaria brizantha* paddock with 1 AU/ha in a continuous grazing system during the experimental period and had free access to water and mineral salt.

Animals that did not have any reproductive diseases; did not receive any type of reproductive procedure (insemination, coverage or embryo collection) 28 days prior to the beginning of the experiment; were clinically healthy at the start of the trial period and had not received any disease treatments for 10 days prior to the study; had not received administration of any hormonal product in the 30 days preceding the study; had a body condition score above 2.5 (scale from 1 to 5, AYRES et. al., Validation of body condition score as a predictor of subcutaneous fat in Nelore (Bos indicus) cows. Livest. Sci. 2009; 123:175-179) were included in the study. To be included in the study, one of the animal's ovaries should have a dominant follicle.

The 40 enrolled cows were assigned to four treatment groups (CB050-1000 - reCGa+β 1000UI, CB050-2500 - reCGa+β 2500UI, Negative Control - saline and Positive Control Novormon 1000UI), so that each group contained 10 animals. The animals in the groups treated with CB050 formulations prepared by concentrating the reCG protein present in the cell culture supernatant by ultrafiltration received a single dose of 1000 IU (CB050-1000) or 2500 IU (CB050-2500) of reCG intramuscularly, eight days after the start of the ovulation synchronization protocol. Animals in the "Positive Control" group received a single dose of 1000 IU/animal, intramuscularly, 8 days after the start of the ovulation synchronization protocol. Animals in the "Negative Control" group received a single dose of 5mL of sterile saline solution.

On the start day of the study (D0), animals were assessed for the body condition score, general health status and reproductive assessment via transrectal ultrasonography. On that same day (D0), the follicular wave emergence synchronization protocol was started. Synchronization consisted of the intramuscular administration of 2mg estradiol benzoate, 530µg sodium cloprostenol together with the insertion of an intravaginal device containing 1g progesterone. Five days after the beginning of the protocol (D5) the cows were homogeneously assigned according to their ages, body condition scores, presence of corpus luteum and follicular population at D0 into 4 experimental groups with 10 animals each, as described above. At that time, animals were assigned into groups, where they received the treatments (1000 IU or 2500 IU of the reCG of the invention; 1000 IU of Novormon or 5 mL of negative control). Eight days later (D8), the intravaginal progesterone device was removed and all animals received intramuscularly 530µg of sodium cloprostenol (PGF) and 1 mg of estradiol cypionate.

Ultrasound assessments (Kai Xin, KX 5100, Xuzhou KaiXin Electronic Instrument Company Ltd, China; Figure 10) were performed in all animals from the study at the following times: selection of animals (D0) and at D8 to assess the number of large follicles, the sum of the number of medium and large follicles, overstimulation rate 1 (sum of large follicles per cow) and overstimulation rate 2 (sum of medium and large follicles per cow).

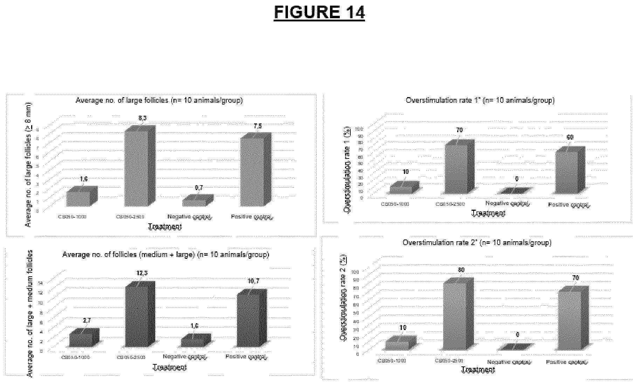

It can be seen in Figure 14 that responses relative to the number of large follicles, the sum of the number of medium and large follicles, and overstimulation rates 1 and 2 associated with treatment with 2500 IU reCG was almost identical to that observed in animals treated with 1000 IU of commercial eCG (Novormon), demonstrating the *in vivo* biological activity of reCG in one of the target species (cows).

## Claims

1. A PROCESS FOR THE PRODUCTION OF A BIOLOGICALLY ACTIVE RECOMBINANT EQUINE CHORIONIC GONADOTROPIN, **characterized in that** it comprises the following steps:
(a) preparing and obtaining DNA sequences encoding reCG α and β chains,
(b) inserting said coding DNA sequences into expression vectors such that said coding DNA sequences are operably linked to gene transcription promoting elements,
(c) genetic engineering a cell line of interest, which comprises the genomic insertion, in the same cell, of a first expression vector of the DNA sequence encoding the reCG α-chain and a second expression vector bearing the DNA sequence which encodes reCG β-chain,
(d) stably co-transfecting the reCG α and β chain expression vectors into the cell line of interest in order to generate mixed cell populations expressing independently said first and second DNA sequences, such that the α and β polypeptide chains are produced as separate molecules in the same cell of said cell line of interest,
(e) isolating cell sub-phenotypes or clones from an initial mixed cell population generated by stable co-transfection of reCG α and β chain expression vectors,
(f) identifying cell clones or sub-phenotypes associated with the production of biologically active reCG forms by assessing the presence of eCG-like biological activity in samples of culture medium conditioned by cell clones representing different cell sub-phenotypes, the eCG-like biological activity being recognized by observing the induction of rapid and vigorous growth of uterine and ovarian tissues in immature rats,
(g) culturing the genetically engineered cell clone expressing the biologically active reCG, and
(h) recovering biologically active reCG from the culture medium conditioned by the genetically engineered cell clone.

2. The PROCESS according to claim 1, **characterized in that** the reCG α and β chain coding sequences are defined in nucleotide sequences of SEQ ID NO.1 and SEQ ID NO.2, respectively.

3. The PROCESS according to claim 1, **characterized in that** the reCG α and β chain coding sequences are cloned intio different expression vectors.

4. The PROCESS according to claim 3, **characterized in that** the the reCG α chain expression vector is plasmid vector pNU1.

5. The PROCESS according to claim 3, **characterized in that** the the reCG β chain expression vector is plasmid vector pcDNA 3.3.

6. The PROCESS according to claim 1, **characterized in that** the cell line of interest is derived from mammalian cells.

7. The PROCESS according to claim 1, **characterized in that** the cell line of interest is CHO-DG44.

8. The PROCESS according to claim 1, **characterized in that** reCG α and β polypeptide chains have the amino acid sequences as defined in SEQ ID NO.3 and SEQ ID NO.4 respectively, are produced by the cell line of interest and secreted thereby in the form of a heterodimer having biological activity in the target species.

9. The PROCESS according to any one of claims 1 to 8, **characterized in that** it provides a reCG expression rate in the range of from 1 to 100 mg/L.

10. The PROCESS according to claim 1, **characterized in that** the purification step is performed by at least one of the following chromatographic methods: ion exchange and affinity chromatography, protein precipitation, ultrafiltration or tangential filtration.

11. A BIOLOGICALLY ACTIVE RECOMBINANT EQUINE CHORIONIC GONADOTROPIN, **characterized in that** it is produced by the process as defined in claims 1 to 10.

12. The BIOLOGICALLY ACTIVE RECOMBINANT EQUINE CHORIONIC GONADOTROPIN according to claim 11, **characterized in that** the reCG α and β polypeptide chains are fully or partially glycosylated.

13. The BIOLOGICALLY ACTIVE RECOMBINANT EQUINE CHORIONIC GONADOTROPIN according to claim 12, **characterized in that** the reCG α and β polypeptide chains are partially glycosylated and contain Gal-(1-4)GIcNAc disaccharide unit as an N-linked and/or O-linked terminal residue, and/or GlcNAc monosaccharide unit as the O-linked terminal residue.

14. A VETERINARY COMPOSITION, **characterized in that** it comprises:
(a) a veterinarily effective amount of the reCG as defined in any one of claims 11 to 13;
(b) optionally additives; and
(c) a pharmaceutically acceptable carrier.

15. USE OF THE BIOLOGICALLY ACTIVE RECOMBINANT EQUINE CHORIONIC GONADOTROPIN, as defined in claim 11, or the veterinary composition as defined in claim 14 containing said gonadotropin, **characterized in that** it is in the manufacture of a medicament for the treatment of veterinary conditions and protocols related to reproduction and ovulation of mammals.

16. USE OF THE BIOLOGICALLY ACTIVE RECOMBINANT EQUINE CHORIONIC GONADOTROPHIN, as defined in claim 11, or the composition as defined in claim 14 containing said gonadotrophin, **characterized in that** it is in the manufacture of a diagnostic kit for veterinary conditions and protocols related to reproduction and ovulation of mammals.

17. USE OF THE BIOLOGICALLY ACTIVE RECOMBINANT EQUINE CHORIONIC GONADOTROFIN, as defined in claim 11, or the composition as defined in claim 14 containing said gonadotropin, **characterized in that** it is in the manufacture of a cell culture supplement.


SEQUENCE LISTING

<110> Universidade de São Paulo USP; Ouro Fino Saúde Animal Participações S. A.

<120> "Biologically active recombinant equine Chorionic Gonadotropin (reCG) and process for obtaining the same, veterinary composition and use"

<140> BR 10 2019 004147 1
<141> 28/02/2019

<160> 4

<210> SEQ ID NO:1
<211> 378
<212> DNA
<213> Equus caballus

<220>
<221> CDS Nucleotide sequence encoding the eCG a chain
<222> (1)...(360)

<400> 1
atg gat tac tac aga aaa cat gca gct gtc atc ctg gcc aca ttg tcc      48
Met Asp Tyr Tyr Arg Lys His Ala Ala Val Ile Leu Ala Thr Leu Ser
 1               5                  10                  15

gtg ttt ctg cat att ctc cat tcc ttt cct gat gga gag ttt aca acg      96
Val Phe Leu His Ile Leu His Ser Phe Pro Asp Gly Glu Phe Thr Thr
            20                  25                  30

cag gat tgc cca gaa tgc aag cta agg gaa aac aag tac ttc ttc aaa     144
Gln Asp Cys Pro Glu Cys Lys Leu Arg Glu Asn Lys Tyr Phe Phe Lys
        35                  40                  45

ctg ggc gtc ccg att tac cag tgt aag ggc tgc tgc ttc tcc aga gcg     192
Leu Gly Val Pro Ile Tyr Gln Cys Lys Gly Cys Cys Phe Ser Arg Ala
    50                  55                  60

tac ccc act cca gca agg tcc agg aag aca atg ttg gtc cca aag aac     240
Tyr Pro Thr Pro Ala Arg Ser Arg Lys Thr Met Leu Val Pro Lys Asn
65                  70                  75                  80

atc acc tca gaa tcc aca tgc tgt gtg gcc aaa gca ttt atc agg gtc     288
Ile Thr Ser Glu Ser Thr Cys Cys Val Ala Lys Ala Phe Ile Arg Val
                85                  90                  95

aca gtg atg gga aac atc aag ttg gag aac cac acc cag tgc tat tgc     336
Thr Val Met Gly Asn Ile Lys Leu Glu Asn His Thr Gln Cys Tyr Cys
            100                 105                 100

agc act tgc tat cac cac aag att taaaggtttcaccaagtg                  378

Ser Thr Cys Tyr His His Lys Ile
   115                 120

<210> SEQ ID NO: 2
<211> 510
<212> DNA
<213> Equus caballus
<221> CDS Nucleotide sequence encoding the eCG ß chain
<222> (1)...(507)

<400> 2
atg gag acg ctc cag ggg ctg ctg ctg tgg atg ctg ctg agt gtt ggc 48
Met Glu Thr Leu Gln Gly Leu Leu Leu Trp Met Leu Leu Ser Val Gly
1               5                   10                  15

ggg gtc tgg gca tcc agg ggg cca ctg cgg cca ctg tgc cgg ccc atc 96
Gly Val Trp Ala Ser Arg Gly Pro Leu Arg Pro Leu Cys Arg Pro Ile
            20                  25                  30

aac gcc act ctg gct gct gag aag gag gcc tgc ccc atc tgc atc acc 144
Asn Ala Thr Leu Ala Ala Glu Lys Glu Ala Cys Pro Ile Cys Ile Thr
        35                  40                  45

ttc acc acc agc atc tgt gcc ggc tac tgc ccc agc atg gtg cgg gtg 192
Phe Thr Thr Ser Ile Cys Ala Gly Tyr Cys Pro Ser Met Val Arg Val
    50                  55                  60

atg cca gct gcc ctg ccg gcc att ccc cag cca gtg tgc acc tac cgt 240
Met Pro Ala Ala Leu Pro Ala Ile Pro Gln Pro Val Cys Thr Tyr Arg
65                  70                  75                  80

gag ctg cgc ttt gct tcc atc cgg ctc ccc ggc tgc ccg cct ggt gtg 288
Glu Leu Arg Phe Ala Ser Ile Arg Leu Pro Gly Cys Pro Pro Gly Val
                85                  90                  95

gac ccc atg gtc tcc ttc ccc gtg gcc ctc agt tgt cac tgc ggg ccc 336
Asp Pro Met Val Ser Phe Pro Val Ala Leu Ser Cys His Cys Gly Pro
            100                 105                 110

tgc cag atc aag acc act gac tgc ggg gtt ttc aga gac cag ccc ttg 384
Cys Gln Ile Lys Thr Thr Asp Cys Gly Val Phe Arg Asp Gln Pro Leu
        115                 120                 125

gcc tgt gcc ccc cag gcc tcc tct tcc tct aag gat ccc cca tcc caa 432
Ala Cys Ala Pro Gln Ala Ser Ser Ser Ser Lys Asp Pro Pro Ser Gln
    130                 135                 140

cct ctc aca tcc aca tcc acc cca act cct ggg gcc agc aga cgt tcc 480
Pro Leu Thr Ser Thr Ser Thr Pro Thr Pro Gly Ala Ser Arg Arg Ser
145                 150                 155                 160

tct cat ccc ctc cca ata aag act tct tga 510
Ser His Pro Leu Pro Ile Lys Thr Ser
                165

<210> SEQ ID NO: 3
<211> 120

<212> PRT
<213> Equus caballus

<221> Protein sequence of eCG a chain

<400> 3
Met Asp Tyr Tyr Arg Lys His Ala Ala Val Ile Leu Ala Thr Leu Ser
1               5                   10                  15

Val Phe Leu His Ile Leu His Ser Phe Pro Asp Gly Glu Phe Thr Thr
            20                  25                  30

Gln Asp Cys Pro Glu Cys Lys Leu Arg Glu Asn Lys Tyr Phe Phe Lys
        35                  40                  45

Leu Gly Val Pro Ile Tyr Gln Cys Lys Gly Cys Cys Phe Ser Arg Ala
    50                  55                  60

Tyr Pro Thr Pro Ala Arg Ser Arg Lys Thr Met Leu Val Pro Lys Asn
65                  70                  75                  80

Ile Thr Ser Glu Ser Thr Cys Cys Val Ala Lys Ala Phe Ile Arg Val
                85                  90                  95

Thr Val Met Gly Asn Ile Lys Leu Glu Asn His Thr Gln Cys Tyr Cys
            100                 105                 110

Ser Thr Cys Tyr His His Lys Ile
        115                 120

<210> SEQ ID NO:4
<211> 169
<212> PRT
<213> Equus caballus

<221> Protein sequence of eCG ß chain

<400> 4
   Met Glu Thr Leu Gln Gly Leu Leu Leu Trp Met Leu Leu Ser Val Gly
   1               5                   10                  15
   Gly Val Trp Ala Ser Arg Gly Pro Leu Arg Pro Leu Cys Arg Pro Ile
               20                  25                  30
   Asn Ala Thr Leu Ala Ala Glu Lys Glu Ala Cys Pro Ile Cys Ile Thr
   35                  40                  45
   Phe Thr Thr Ser Ile Cys Ala Gly Tyr Cys Pro Ser Met Val Arg Val
       50                  55                  60
   Met Pro Ala Ala Leu Pro Ala Ile Pro Gln Pro Val Cys Thr Tyr Arg
   65                  70                  75                  80
   Glu Leu Arg Phe Ala Ser Ile Arg Leu Pro Gly Cys Pro Pro Gly Val
                   85                  90                  95
   Asp Pro Met Val Ser Phe Pro Val Ala Leu Ser Cys His Cys Gly Pro
               100                 105                 110
   Cys Gln Ile Lys Thr Thr Asp Cys Gly Val Phe Arg Asp Gln Pro Leu
           115                 120                 125
   Ala Cys Ala Pro Gln Ala Ser Ser Ser Ser Lys Asp Pro Pro Ser Gln
       130                 135                 140
   Pro Leu Thr Ser Thr Ser Thr Pro Thr Pro Gly Ala Ser Arg Arg Ser
   145                 150                 155                 160
   Ser His Pro Leu Pro Ile Lys Thr Ser
                   165